# EUROPÄISCHE PATENTANMELDUNG

(11) **EP 3 705 107 A1**
(43) Veröffentlichungstag der Anmeldung: **09.09.2020**
(21) Anmeldenummer: 20161430.2
(22) Anmeldetag: 06.03.2020
(51) Int. Cl.: A61H 39/00, A61N 5/06

(54) **HALTERUNG FÜR DIE LICHT-AKUPUNKTUR**

(30) Priorität: 07.03.2019 DE 102019105774
(71) Anmelder: Cayemitte-Rückner, Naomie, 22297 Hamburg (DE)
(72) Erfinder: Cayemitte-Rückner, Naomie, 22297 Hamburg (DE)
(74) Vertreter: Jaeschke, Rainer

(57) **Zusammenfassung**

Die Erfindung betrifft eine Halterung für die Licht-Akupunktur am Kopf, welche einen Halteabschnitt aufweist, der sich in der angelegten Lage zumindest teilweise über das Schläfenbein oberhalb eines Ohrs erstreckt. Gemäß der Erfindung wird vorgeschlagen, dass der Halteabschnitt eine oder mehrere Öffnungen aufweist, durch die jeweils ein Lichtwellenleiter steckbar ist derart, dass er mit seiner im Betrieb leuchtenden Stirnseite an dem Kopf im Bereich des Schläfenbeins anliegt.

## Beschreibung

Die Erfindung betrifft eine Halterung für die Licht-Akupunktur am Kopf, welche einen Halteabschnitt aufweist, der sich in der angelegten Lage zumindest teilweise über das Schläfenbein oberhalb eines Ohrs eines Menschen erstreckt.

Die heilende Wirkung von der herkömmlichen Akupunktur mit Nadeln nach dem Wissen der traditionellen chinesischen Medizin ist allgemein bekannt. Hier werden durch Nadeln die Reflexzonen an bestimmten Bereichen des Körpers stimuliert, so dass beispielsweise Entgiftungsprozesse beschleunigt und das Immunsystem angeregt werden.

Weiterhin ist es bekannt, diese Reflexzonen nicht nur durch mechanische Nadeln sondern auch durch Licht zu stimulieren. Hierbei wird als Licht in der Regel ein Laser-Licht verwendet wird, um die gewünschte Stimulation zu bewirken.

Die Licht-Akupunktur oder die traditionelle Akupunktur haben sich insbesondere bei chronischen Beschwerden bewährt. Das Problem bei der Licht-Akupunktur ist darin zu sehen, dass das Licht über eine bestimmte Zeit auf die ausgewählte Reflexzone einwirken muss. Dies erfordert vom behandelnden Arzt einige Geschicklichkeit und hinreichende Kenntnisse. Auch muss die Lichtquelle dann für eine bestimmte Zeit auf die Reflexzone gehalten werden, um diese zu stimulieren. Hierfür müssen der Arzt und der behandelte Patient ruhig bleiben. Für die Laser-Licht-Akupunktur am Körper gibt es Mehrkanal-Lasergeräte, bei denen die Lichtaustrittsquelle, beispielsweise ein Lichtwellenleiter, mit Hilfe eines Klebebands über den Akupunkturpunkt fixiert wird. Dies ist am Kopf wegen der Haare nicht ohne weiteres möglich.

Erfahrungen haben gezeigt, dass durch die Stimulation der Reflexzonen im Bereich des Ohrs gute Wirkungen erzielt worden sind. Es ist auch bekannt, dass am Kopf eine Vielzahl von Reflexzonen vorhanden sind, auf denen verschiedene Körperregionen abgebildet sind. Viele der zu behandelnden Reflexzonen oder Akupunkturpunkte befinden sich teilweise hinter dem Ohr und teilweise oberhalb des Ohrs. Im Bereich des gesamten Schläfenbeins befinden sich mehrere für diese Behandlung effektive Reflexzonen oder Akupunkturpunkte. Eine Behandlung dieser Reflexzonen ist die Mastoid-Akupunktur, die von der Anmelderin eingehend untersucht worden ist.

Während das Auffinden der bekannten Reflexzonen keine besonderen Schwierigkeiten bereitet, ist das Beleuchten der ausgewählten Reflexzone für die Licht-Akupunktur über einen längeren Zeitraum für die behandelnde Person recht mühsam. Der Erfindung liegt daher die Aufgabe zugrunde, eine Halterung der eingangs geschilderten Art so auszubilden, dass die Licht-Akupunktur am Kopf bequem durchführbar ist.

Die Aufgabe wird gemäß der Erfindung dadurch gelöst, dass der Halteabschnitt eine oder mehrere Öffnungen aufweist, durch die jeweils ein Lichtwellenleiter steckbar ist derart, dass er mit seiner im Betrieb leuchtenden Stirnseite an dem Kopf im Bereich des Schläfenbeins anliegt. Durch den im Allgemeinen flexiblen Lichtwellenleiter ist es möglich, einen eng begrenzten Lichtstrahl unmittelbar auf die ausgewählte Reflexzone am Kopf zu leiten. Durch die Flexibilität des Lichtwellenleiters kann der behandelnde Arzt das Licht bequem an die gewünschte Stelle bzw. gewünschten Stellen leiten. Die Halterung hält den Halteabschnitt in einer vorgegebenen Weise über dem Ohr, so dass die Lichtwellenleiter durch die Öffnungen zu der gewünschten Reflexzone geführt werden können. Sobald der Lichtwellenleiter die gewünschte Reflexzone beleuchtet, kann der behandelnde Arzt einen weiteren Reflexpunkt auswählen.

Die Halterung dient letztlich dazu, mehrere Lichtwellenleiter für die Stimulation von mehreren Reflexzonen im Bereich des Schläfenbeins zu fixieren derart, dass das Licht auch über einen längeren Zeitraum von beispielsweise mehreren Minuten auf die Reflexzone einwirken kann. Unerwünschte Verschiebungen werden durch diese Halterung zuverlässig vermieden. Es werden demnach tatsächlich nur die ausgewählten Akupunkturpunkte stimuliert. Weiterhin kann der behandelnde Arzt nach dem Anlegen der Halterung den Patienten in Ruhe lassen.

Grundsätzlich kann die Halterung einen Halteabschnitt das Schläfenbein auf einer Seite aufweisen. Es ist jedoch zweckmäßig, wenn die Halterung mit zwei Halteabschnitten versehen ist, die sich jeweils in der angelegten Lage zumindest teilweise über das Schäfenbein auf beiden Seiten des Kopfs erstrecken. Hierdurch kann eine gleichzeitige Behandlung auf beiden Seiten des Kopfes erfolgen.

Durch die Ausbildung der Halteabschnitte ist es möglich, die Lichtwellenleiter sehr gut im unmittelbaren Kontakt zu der Kopfhaut am Kopf zu halten. Es ist jedoch günstig, wenn die Lichtwellenleiter in den Öffnungen hin- und herverschiebbar gehalten sind. Dadurch ist es möglich, die Halterungen und die dem Kopf zugwandte Länge des Lichtwellenleiters an unterschiedliche Kopfformen oder unterschiedliche Gegebenheiten im Bereich der Reflexzonen anzupassen.

Gemäß einer weitergehenden Ausführungsform der Erfindung ist vorgesehen, dass die Lichtwellenleiter unter elastischer Aufweitung der jeweiligen Öffnung in dieser geführt und am Halteabschnitt gehalten sind. Hierdurch wird mit einfachen Mitteln eine relativ stramme Halterung der Lichtwellenleiter am Halteabschnitt bewirkt, so dass ein einmal eingestellter Lichtwellenleiter sich nicht ohne Weiteres in der Öffnung verschiebt. Das Licht kann somit sehr gut direkt auf die Kopfhaut und somit auf die Reflexzone einwirken.

Es ist günstig, wenn die Öffnungen entsprechend der Reflexpunkte für die Mastoid-Akupunktur in dem Halteabschnitt angeordnet sind. Es hat sich herausgestellt, dass die Reflexpunkte im Wesentlichen unabhängig von dem individuellen Menschen sich stets an der gleichen Lage relativ zum Ohransatz befinden. Es ist daher möglich, dass der Halteabschnitt ein vorgegebenes Raster an Öffnungen aufweist, durch die die Lichtwellenleiter geführt werden können. Der behandelnde Arzt braucht dann nur noch die betreffenden Öffnungen auszuwählen, um eine Stimulation der gewünschten Reflexzone mit dem Lichtwellenleiter zu erreichen.

Besonders zweckmäßig ist es, wenn die Halterung so ausgebildet ist, dass die Öffnungen in den Halteabschnitten entsprechend den zu behandelnden und zu stimulierenden Reflexzonen hinter und/oder über dem Ohr angeordnet sind. Es ist daher vorgesehen, dass die Öffnungen in den Halteabschnitten für die Behandlung eines spezifischen Krankheitsbildes angeordnet sind.

So kann es zweckmäßig sein, dass eine Halterung und die Anordnung der Öffnungen in den Halteabschnitten für die Behandlung von beispielsweise Haltungsproblemen und eine andere Halterung für die Behandlung von beispielsweise Rückenschmerzen ausgebildet ist. Die Handhabung wird damit vereinfacht. Insbesondere wird eine versehentliche Stimulierung von falschen Reflexzonen vermieden, da ein Einführen eines Lichtwellenleiters in die verkehrte Öffnung der Halterung verhindert beziehungsweise gar nicht möglich ist. Die Halterung weist nur noch solche Öffnungen für Lichtwellenleiter auf, die für die Behandlung eines Problems benötigt werden. Die Halterabschnitte sind daher als Schablonen ausgebildet, die für jede mögliche oder gewünschte Behandlung nur die erforderlichen Öffnungen aufweist.

Da die Lage der verschiedenen Reflexzonen am und über und hinter dem Ohr für jeden Menschen nahezu gleich ist, können die Halterungen unabhängig von dem individuellen Patienten vorgefertigt werden. Die Lage der Öffnungen für die Stimulierung der ausgewählten Reflexzonen muss dann nur noch relativ zum Ohr des Patienten ausgerichtet werden.

Die Anmelderin hat herausgefunden, dass die Lage der Reflexzonen relativ zum oberen und vorderen Ohransatz für jeden Menschen nahezu identisch und unabhängig von der Größe des Menschen beziehungsweise dessen Kopfs sind. Es ist daher zweckmäßig, wenn die Halterung oder die Haltabschnitte und die betreffenden Schablonen mit den Öffnungen eine Markierung aufweisen, mit der die Halteabschnitte beziehungsweise die Schablonen relativ zu dem oberen und vorderen Ohransatz ausgerichtet werden. Es kann beispielsweise vorgesehen werden, dass der Halteabschnitt eine Markierung aufweist, die in der wirksamen Lage vertikal über dem vorderen und oberen Ohransatz liegt. Die horizontale Ausrichtung der Halteabschnitte wird durch einen unteren Rand der Halterung oder der Halteabschnitte bewirkt, der auf der oberen Ohransatzlinie aufliegt. Der Rand kann beispielsweise 1,5 cm bis 3 cm lang sein. Die Öffnungen der Halteabschnitte beziehungsweise der Schablonen sind dann entsprechend der ausgewählten Reflexzonen ausgerichtet.

Aus welchem Material der Halteabschnitt oder die Halteabschnitte bestehen, ist grundsätzlich beliebig. Günstig ist es jedoch, wenn der Halteabschnitt oder die Halteabschnitte aus einem Kunststoffmaterial und insbesondere aus Silikon bestehen. Silikon hat elastische und auch flexible Eigenschaften, so dass es sich gut an den Kopf des Patienten anlegt. Auch kann Silikon gut gereinigt werden. Grundsätzlich kann aber auch ein anderes elastisches und/oder flexibles Kunststoffmaterial verwendet werden.

Zweckmäßig ist es auch, wenn die Halteabschnitte aus einem durchsichtigen Material bestehen. Dann kann der behandelnde Arzt das richtige Anliegen des Lichtwellenleiters auf der Kopfhaut bei angelegter Halterung kontrollieren.

Alternativ ist es möglich, dass der Halteabschnitt oder die Halteabschnitte aus einem textilen Material bestehen. Hier können die Öffnungen durch die Maschen gebildet werden, so dass jede gewünschte Reflexzone im Bereich des Kopfes erreicht werden kann.

In jedem Fall ist es günstig, wenn der Halteabschnitt oder die Halteabschnitte an der Halterung verschiebbar gehalten sind. Hierdurch lässt sich die Halterung gut an unterschiedliche Kopfformen oder Kopfgrößen anpassen.

Weiterhin ist es zweckmäßig, wenn der Halteabschnitt oder die Halteabschnitte lösbar mit der Halterung verbunden sind. Hierdurch wird die Reinigung der Halterung erleichtert, da lediglich die gelösten Halteabschnitte gereinigt und desinfiziert zu werden brauchen.

Es kann vorgesehen werden, dass sich ein Halteabschnitt zumindest teilweise hinter einem Ohr erstreckt. Hierdurch wird erreicht, dass die Reflexzonen des Schläfenbeins, die von der Ohrmuschel überdeckt werden, mit Licht beaufschlagt werden können. Der Halteabschnitt weist dort ebenfalls Durchbrechungen für Lichtwellenleiter auf.

Es kann auch zweckmäßig sein, wenn der Halteabschnitt einen Fortsatz mit Durchbrechungen für Lichtwellenleiter aufweist, der den Bereich unter der Ohrmuschel am hinteren Ohransatz abdeckt. Dieser Fortsatz kann flexibel mit dem Halteabschnitt verbunden und an diesen angeformt sein. Es ist aber auch möglich, dass der Fortsatz im Bereich hinter dem Ohr als eine nach unten weisende Ausbuchtung ausgebildet ist, der in der angelegten Lage unter der Ohrmuschel am hinteren Ohransatz am Schläfenbein anliegt. Dadurch werden mit einfachen Mitteln auch die Reflexzonen für die Mastoid-Akupunktur am Schläfenbein überdeckt und mit Lichtwellenleitern erreichbar, welche hinter dem Ohransatz liegen.

Die Halterung kann beispielsweise als Haube ausgebildet sein, um den gesamten Kopfbereich abzudecken derart, dass die Lichtwellenleiter durch entsprechend angeordnete Öffnungen zu verschiedenen Reflexpunkten am Kopf führbar sind. Dadurch können auch Reflexzonen außerhalb des Schläfenbeins mit Licht stimuliert werden.

Besonders zweckmäßig ist es jedoch, wenn die Halterung als Kopfband ausgebildet ist. Dieses Kopfband kann in der Weite verstellbar sein, so dass es in einfacher Weise an den Kopfumfang angepasst werden kann. Das Kopfband erstreckt sich somit über die Stirn und den Hinterkopf des Patienten, so dass die Halteabschnitte sicher im Bereich oberhalb des Ohrs gehalten werden. Das Kopfband kann beispielsweise gummielastisch ausgebildet sein oder ein längenverstellbares Kunststoffband umfassen.

Gemäß einer anderen Ausführungsform der Erfindung ist vorgesehen, dass die Halterung als Bügel ausgebildet ist, der sich über den Kopf erstreckt und an seinen freien Enden die Halteabschnitte trägt. Der Bügel kann die Halteabschnitte elastisch aber ausreichend fest an das Schläfenbein oberhalb des Ohrs andrücken. Weiterhin kann der Bügel längenverstellbar ausgebildet sein, so dass er ohne Weiteres an unterschiedliche Kopfgrößen anpassbar ist.

Die Lichtwellenleiter sind in der Regel elastisch und biegsam ausgebildet. Es ist somit in einfacher Weise möglich, das leuchtende Stirnende unmittelbar auf der Kopfhaut zu platzieren. Durch den vorhandenen Abstand zwischen dem Halteabschnitt und der Kopfhaut ist es möglich, beispielsweise störende Haare zu umgehen.

Die anderen Enden der Lichtwellenleiter können zusammengeführt und einer gemeinsamen Lichteinspeisungsquelle zugeführt werden. Es können Hochleistungs-LED verwendet werden. Es hat sich gezeigt, dass Hochleistungs-LED eine ausreichende aber auch nicht zu große Lichtstärke erzeugen können, um die Reflexzonen oder Reflexpunkte zu stimulieren. Durch die Verwendung von LED ist der Gebrauch der Halterung auch ungefährlich.

Es kommt bei der Behandlung auf die Stimulierung der Knochenhaut an. Es ist daher wesentlich, dass das einwirkende Licht nur bis zur Knochenhaut, dem Periost, am Schädel eindringt. Die Lichtstärke der eingesetzten Lichtquelle ist demnach so bemessen, dass ein Eindringen des Lichts nur bis zur Knochenhaut am Schädel bewirkt wird. Das Licht einer stärkeren Lichtquelle, beispielsweise einer Laserlichtquelle, würde bis in das Gehirn endringen und dort auf die Gehirnzellen unmittelbar einwirken, was nicht erwünscht ist. Es erfolgt durch das nur auf das Periost einwirkende Licht eine Reizung oder Stimulierung der betreffenden Reflexzonen, die sich auf dem Periost befinden und auf denen bestimmte Körperregionen abgebildet sind.

Durch eine solche Halterung beziehungsweise einen solchen Halteabschnitt oder eine Schablone kann die Stimulierung des betreffenden Mikrosystems des Körpers erfolgen. Die Reflexzonen oder Reflexpunkte des gewünschten Mikrosystems werden durch die vorgegebenen Öffnungen in den Halteabschnitten zuverlässig gefunden und können gezielt mit Licht gereizt werden. Es kann mit der Halterung gemäß der Erfindung eine LED-Lichtakupunktur und somit eine Periost-Akupunktur von vorbestimmten Reflexzonen im Bereich des Schläfenbeins für einen längeren Zeitraum für den behandelnden Arzt bequem und sicher durchgeführt werden.

Weiterhin kann vorgesehen werden, dass das Licht mit unterschiedlichen Wellenlängen strahlt. Diese Wellenlängen können entweder frei wählbar oder automatisch verändert werden. Auch kann ein gepulstes Licht eingesetzt werden. Letztlich hängt dies vom behandelnden Arzt ab.

Die Erfindung wird im Folgenden anhand der schematischen Zeichnung näher erläutert. Es zeigen:

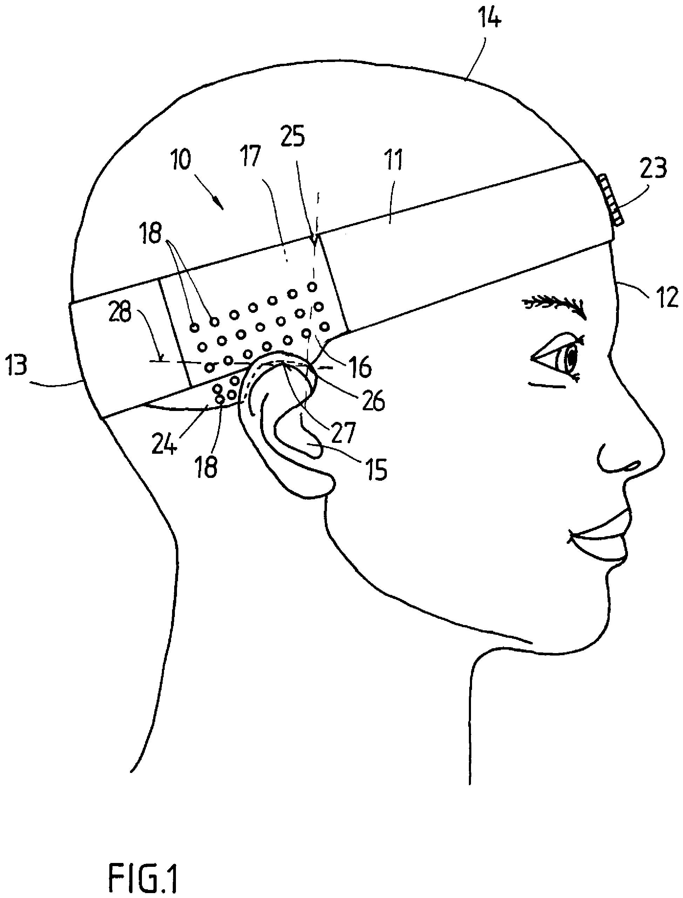

- Fig. 1:: Die Seitenansicht eines Kopfes mit angelegter Halterung gemäß der Erfindung,

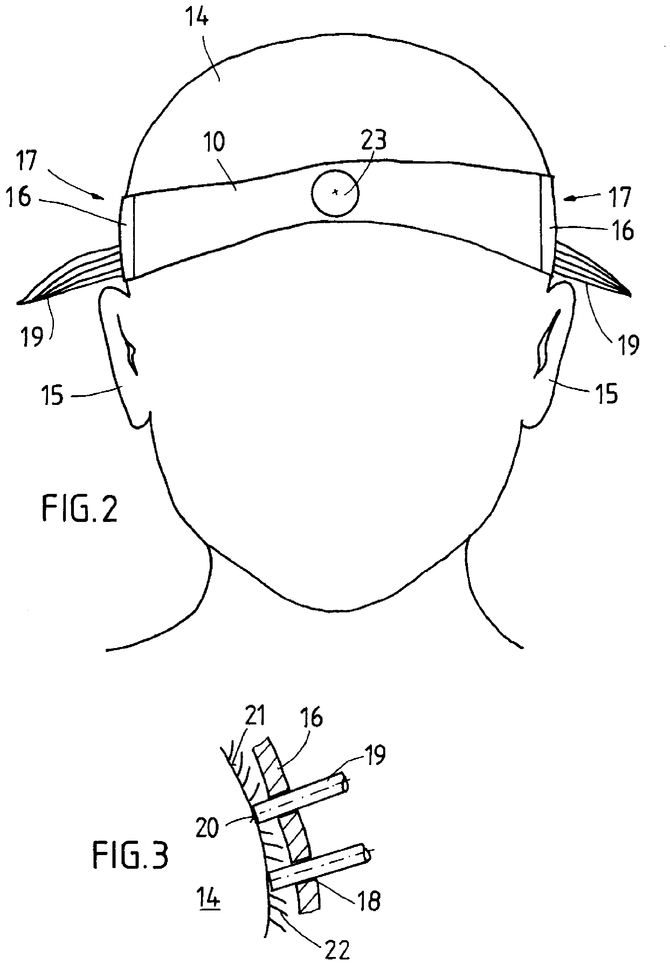

- Fig. 2:: Die Frontansicht eines Kopfes mit angelegter Halterung und
- Fig. 3:: Einen Schnitt durch den Halteabschnitt im Bereich der Öffnungen.

Die in der Zeichnung dargestellte Halterung 10 für die Licht-Akupunktur ist als Kopfband 11 ausgebildet, das sich über den Stirnbereich 12 und den Hinterkopf 13 des Kopfs 14 eines Menschen erstreckt. Das Kopfband 11 hat demnach in etwa das Aussehen eines Stirnbands.

Im Bereich oberhalb eines Ohrs 15 weist die Halterung 10 auf jeder Seite einen Halteabschnitt 16 auf, der sich über das Schläfenbein 17 oberhalb des Ohrs 15 erstreckt. Dadurch wird das Schläfenbein 17 des Kopfes 14 zumindest teilweise überdeckt. In dem Halteabschnitt 16 sind Öffnungen 18 vorgesehen, durch die Lichtwellenleiter 19 passen. Im Einzelnen ist die Anordnung so getroffen, dass die Lichtwellenleiter 19 relativ stramm in den Öffnungen 18 gehalten sind, so dass sie sich entgegen der Reibungskraft hin- und herverschieben lassen. Dadurch wird erreicht, dass die Lichtwellenleiter 19 bzw. deren freien und bei Betrieb leuchtenden Stirnseiten 20 gut an die Kopfhaut 21 des Kopfs 14 anlegen lassen. Durch die reibende Führung des Lichtwellenleiters 19 in der zugeordneten Öffnung 18 wird ein Zurückbewegen des Lichtwellenleiters beispielsweise aufgrund einer Kopfbewegung zuverlässig verhindert.

Die Öffnungen 18 sind am Halteabschnitt 16 so angeordnet, dass sie in der angelegten Lage im Wesentlichen über den bekannten Reflexzonen der Mastoid-Akupunktur am Schläfenbein 17 des Kopfs 14 und insbesondere oberhalb und teilweise hinter dem Ohr 15 eines Menschen liegen. Der Lichtwellenleiter 19 wird senkrecht zu dem Halteabschnitt 16 in den Öffnungen 18 gehalten, so dass das freie Stirnende 20 sicher auf der Kopfhaut 21 zum Anliegen kommt. Der Durchmesser der Lichtwellenleiter ist so gewählt, dass der Lichtwellenleiter gut zwischen den Haaren 22 unmittelbar auf der Kopfhaut 21 platziert werden kann. Das Licht kann somit direkt auf die Kopfhaut und somit auf die Reflexzonen am Schläfenbein 17 einwirken.

Der Durchmesser der Lichtwellenleiter 19 beträgt beispielsweise 1,5 mm bis 5 mm und insbesondere 2 mm bis 3 mm. Die freien Stirnenden 20 der Lichtwellenleiter 19 können plan ausgebildet sein. Für ein angenehmes Tragen können die Stirnenden 20 auch abgerundet ausgebildet sein. Zumindest ist es zweckmäßig, wenn die Kanten abgerundet sind, um ein unangenehmes Andrücken an die Kopfhaut zu vermeiden.

Bei der gezeigten Ausführungsform ist das Kopfband 11 fest mit den Halteabschnitten 16 verbunden. Insbesondere ist vorgesehen, dass das Kopfband 11 und die Halteabschnitte 16 auf beiden Seiten aus einem Kunststoffmaterial bestehen. Das Kunststoffmaterial weist insbesondere im Bereich der Halteabschnitte 16 eine Dicke auf, die ausreicht, den durchgesteckten Lichtwellenleiter 19 in den Öffnungen 18 sicher zu führen. Ein Abknicken oder ein unerwünschter schräger Verlauf des Lichtwellenleiters 19 relativ zu dem Halteabschnitt 16 wird dadurch vermieden. Die Dicke der Halteabschnitte 16 kann beispielsweise 3 bis 8 mm betragen.

Das Kopfband kann aber auch lösbar mit den Halteabschnitten 16 verbunden sein. Dann kann das Kopfband 11 aus einem gummielastischen Material bestehen, so dass die Halteabschnitte gut und unabhängig von der tatsächlichen Kopfgröße in der gewünschten Lage über dem Ohr gehalten werden.

Es kann aber auch vorgesehen werden, dass bei einer einstückigen Ausbildung der Halterung 10 ein Verstellmechanismus 23 vorgesehen ist, durch den die Weite des Kopfbands 11 verstellt werden kann. Solche Verstellmechanismen sind grundsätzlich bekannt und bedürfen daher keiner weiteren Erläuterung.

Grundsätzlich ist es vorgesehen, dass die Halteabschnitte 16 oberhalb eines Ohrs 15 verlaufen. Es kann aber auch vorgesehen werden, dass die Halteabschnitte 16 im Bereich des hinteren Ohransatzes jeweils einen Fortsatz 24 mit Durchbrechungen 18 aufweisen, der in der angelegten Lage zumindest teilweise unterhalb der Ohrmuschel am Schläfenbein 17 anliegt. Die Ausrichtung der Lichtwellenleiter 19 relativ zu den Reflexzonen am gesamten Schläfenbein wird dadurch möglich.

Die Lichtwellenleiter können einen Durchmesser von 1,5 mm bis 5 mm und insbesondere von 2 mm bis 3 mm aufweisen, so dass sie gut zwischen die Haare zu der Kopfhaut geführt werden können. Das an dem freien Stirnende 20 austretende Licht kann somit direkt auf die Kopfhaut 21 und auf die Akupunkturpunkte einwirken. An einem Halteabschnitt 16 können mehrere Lichtwellenleiter 19 zum Behandeln von mehreren Reflexzonen angeordnet sein. Es ist zweckmäßig, wenn die Lichtwellenleiter 19 in einem Abstand zu dem Halteabschnitt 16 gebündelt und zusammengeführt zu einer nicht gezeigten Lichtquelle geführt werden. Die Lichtquelle kann sich in einem Abstand von beispielsweise 0,8 bis 2,0 Metern zu der Halterung befinden, so dass ein bequemes Anlegen und Tragen der Halterung 10 möglich ist.

Wie der Lichtwellenleiter im Einzelnen ausgebildet ist, ist grundsätzlich bekannt und bedarf daher keiner weiteren Erläuterung. Als Lichtquelle können eine oder mehrere Hochleistungs-LED verwendet werden, die Licht mit unterschiedlichen Wellenlängen ausstrahlen können. Damit ist es möglich, die Reflexzonen mit unterschiedlichen Wellenlängen zu stimulieren.

Die Halterung ist in einfacher Weise am Kopf eines Menschen anzulegen. Dies kann entweder durch geschultes Fachpersonal oder aber durch den Patienten selbst erfolgen. Das Ausrichten der Lichtwellenleiter auf den Reflexzonen kann individuell erfolgen. Hier kann vorgesehen werden, dass für einen Patienten eine Halterung mit Öffnungen an den für ihn relevanten Reflexzonen vorgesehen ist. Es kann aber auch vorgesehen werden, dass die Öffnungen 18 entlang eines Rasters angeordnet sind, so dass der Lichtwellenleiter bei angelegter Halterung durch die gewünschte Öffnung zu der gewünschten Reflexzone geführt wird. Insgesamt gelingt es dadurch, zum einen eine ausreichende Lichtmenge unmittelbar zu der Reflexzone zu leiten. Weiterhin ist es möglich, dass der Lichtwellenleiter über einen längeren Zeitraum von beispielsweise 5 bis 20 Minuten und insbesondere 7 bis 15 Minuten auf der gleichen Reflexzone bleibt, so dass die Reflexzone ausreichend lange stimuliert wird.

Es kann aber auch vorgesehen werden, dass die Halteabschnitte 16 vorgefertigte Öffnungen 18 für die Behandlung bestimmter Krankheitsbilder aufweisen. Die Öffnungen liegen dann in der angelegten Lage der Halterung über den ausgewählten Reflexpunkten für die Behandlung des betreffenden Krankheitsbilds. Damit die Halterung und die Halteabschnitte 16 und somit die Öffnungen 18 richtig zum Ohr 15 des Patienten ausgerichtet werden können, weist die Halterung 10 eine Markierung 25 auf, die in der richtigen Position der Halterung 10 vertikal beziehungsweise kranial über dem vorderen und oberen Ohransatzpunkt 26 ausgerichtet ist. Die Markierung 25 kann als Kerbe ausgebildet sein.

Die horizontale Ausrichtung kann über einen horizontalen unteren Rand 27 der Halterung oder der Halteabschnitte 16 erfolgen, der sich in der richtigen Position der Halterung 10 auf der oberen horizontalen Ohransatzlinie 28 abstützt. Damit ist eine exakte Ausrichtung der Halterung beziehungsweise deren Halteabschnitte 16 relativ zum Ohr 15 möglich. Durch die elastische Ausgestaltung des Bands 11 kann die Halterung bei gleicher Ausrichtung der Halteabschnitte 16 relativ zu dem jeweils zugeordneten Ohr an unterschiedliche Kopfgrößen angepasst werden.

## Patentansprüche

1. Halterung (10) für die Licht-Akupunktur am Kopf (14), welche einen Halteabschnitt (16) aufweist, der sich in der angelegten Lage zumindest teilweise über das Schläfenbein (17) oberhalb eines Ohrs (15) erstreckt, **dadurch gekennzeichnet, dass** der Halteabschnitt (16) eine oder mehrere Öffnungen (18) aufweist, durch die jeweils ein Lichtwellenleiter (19) steckbar ist derart, dass er mit seiner im Betrieb leuchtenden Stirnseite (20) an dem Kopf (14) im Bereich des Schläfenbeins (17) anliegt.

2. Halterung nach Anspruch 1, **dadurch gekennzeichnet, dass** zwei Halteabschnitte (16) vorhanden sind, die sich jeweils in der angelegten Lage zumindest teilweise über das Schläfenbein auf beiden Seiten des Kopfs (14) erstrecken.

3. Halterung nach Anspruch 1 oder 2, **dadurch gekennzeichnet, dass** die Lichtwellenleiter (19) in den Öffnungen (18) hin- und herverschiebbar gehalten sind.

4. Halterung nach einem der Ansprüche 1 bis 3, **dadurch gekennzeichnet, dass** die Lichtwellenleiter (19) unter elastischer Aufweitung der jeweiligen Öffnung (18) oder unter elastischem Zusammendrücken des Lichtwellenleiters reibend in der Öffnung (18) geführt und somit am Halteabschnitt (16) gehalten sind.

5. Halterung nach einem der Ansprüche 1 bis 4, **dadurch gekennzeichnet, dass** die Öffnungen (18) entsprechend der Reflexpunkte für die Mastoid-Akupunktur in dem Halteabschnitt (16) angeordnet sind.

6. Halterung nach einem der Ansprüche 1 bis 5, **dadurch gekennzeichnet, dass** der Halteabschnitt oder die Haltabschnitte (16) aus einem Kunststoffmaterial und insbesondere aus Silikon bestehen.

7. Halterung nach einem der Ansprüche 1 bis 6, **dadurch gekennzeichnet, dass** der Halteabschnitt oder die Haltabschnitte (16) aus einem durchsichtigen Material bestehen.

8. Halterung nach einem der Ansprüche 1 bis 7, **dadurch gekennzeichnet, dass** der Halteabschnitt oder die Haltabschnitte (16) an der Halterung (10) verschiebbar gehalten sind.

9. Halterung nach einem der Ansprüche 1 bis 8, **dadurch gekennzeichnet, dass** der Halteabschnitt oder die Halteabschnitte (16) lösbar mit der Halterung (10) verbunden sind.

10. Halterung nach einem der Ansprüche 1 bis 9, **dadurch gekennzeichnet, dass** der Halteabschnitt (16) einen Fortsatz mit Durchbrechungen (18) für Lichtwellenleiter (19) aufweist, der den Bereich unter der Ohrmuschel am hinteren Ohransatz abdeckt.

11. Halterung nach einem der Ansprüche 1 bis 10, **dadurch gekennzeichnet, dass** sie als Haube ausgebildet ist, um den gesamten Kopfbereich abzudecken derart, dass die Lichtwellenleiter durch entsprechend angeordnete Öffnungen zu verschiedenen Reflexpunkten am Kopf führbar sind.

12. Halterung nach einem der Ansprüche 1 bis 10, **dadurch gekennzeichnet, dass** sie als Kopfband ausgebildet ist.
